(19) Europäisches Patentamt
European Patent Office
Office européen des brevets

(11) Veröffentlichungsnummer: **0 083 744**
**A2**

(12) # EUROPÄISCHE PATENTANMELDUNG

(21) Anmeldenummer: 82111462.6

(22) Anmeldetag: 10.12.82

(51) Int. Cl.³: C 07 D 249/08
C 07 D 233/12, A 01 N 47/24

(30) Priorität: 23.12.81 DE 3150984

(43) Veröffentlichungstag der Anmeldung: 20.07.83 Patentblatt 83/29

(84) Benannte Vertragsstaaten: AT BE CH DE FR GB IT LI NL

(71) Anmelder: BAYER AG
Zentralbereich Patente, Marken und Lizenzen
D-5090 Leverkusen 1, Bayerwerk(DE)

(72) Erfinder: Schmetzer, Johannes, Dr.
Jakob-Böhme-Strasse 21
D-5000 Koeln 80(DE)

(72) Erfinder: Stetter, Jörg, Dr.
Gellertweg 4
D-5600 Wuppertal 1(DE)

(72) Erfinder: Hammann, Ingeborg, Dr.
Lutherstrasse 22
D-4330 Mülheim/Ruhr(DE)

(72) Erfinder: Homeyer, Bernhard, Dr.
Obere Strasse 28
D-5090 Leverkusen 3(DE)

(54) **Oximcarbamate, Verfahren zu ihrer Herstellung, ihre Verwendung als Schädlingsbekämpfungsmittel, sowie Zwischenprodukte zu ihrer Herstellung und Verfahren zur Herstellung dieser Zwischenprodukte.**

(57) Die vorliegende Erfindung betrifft die neuen Oximcarbamate der Formel (I)

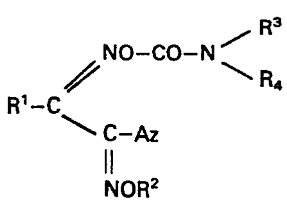

in welcher
$R^1$, $R^2$, $R^3$, $R^4$, Az die in der Beschreibung angegebene Bedeutung besitzen. Sie werden erhalten, indem man

a) Oxime der Formel (II)

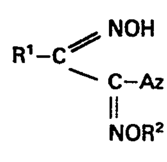

mit Isocyanaten der Formel

$R^4$ NCO

oder mit Carbamoylhalogeniden der Formel (IV)

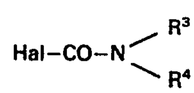

./...

EP 0 083 744 A2

Croydon Printing Company Ltd.

umsetzt oder mit Phosgen und anschließend mit Aminen

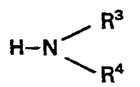

umsetzt, oder
gegebenenfalls die nach den obengenannten Verfahren erhältlichen Oximcarbamate der Formel (VI)

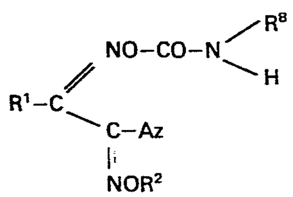

mit Sulfenchloriden der Formel

$R^5-S-Cl$

umsetzt.

Die Oximcarbamate der Formel (I) lassen sich als Insektizide, Akarizide und Nematizide verwenden.

BAYER AKTIENGESELLSCHAFT
5090 Leverkusen, Bayerwerk
Zentralbereich
Patente, Marken und Lizenzen
Rt/bc/c

BEZEICHNUNG GEÄNDERT
siehe Titelseite

# Oximcarbamate, Verfahren zu ihrer Herstellung und ihre Verwendung als Schädlingsbekämpfungsmittel

Die vorliegende Erfindung betrifft neue Oximcarbamate, Verfahren zu ihrer Herstellung, ihre Verwendung als Schädlingsbekämpfungsmittel, sowie Zwischenprodukte zu ihrer Herstellung und Verfahren zur Herstellung dieser Zwischenprodukte.

Es sind bereits Oximcarbamate wie 3,3-Dimethyl-2-oximino-N-methylcarbamoyl-1-(pyrazol-1-yl)-butan bekannt. Doch befriedigt deren Wirkung vor allem bei niedrigen Aufwandkonzentrationen nicht immer voll (vgl. DE-OS 2 613 167).

1)

Es wurden die neuen Oximcarbamate der Formel (I) gefunden

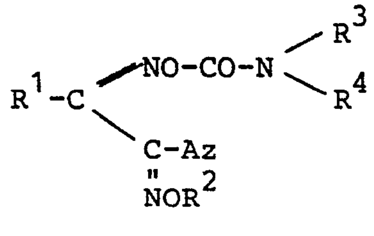

(I)

in welcher

Az für einen gegebenenfalls substituierten Azolylrest steht,

$R^1$ für gegebenenfalls substituiertes Alkyl steht,

$R^2$ für Alkyl, Halogenalkyl, Cycloalkyl, Alkenyl, Alkinyl, Alkyloxyalkyl, Alkylthioalkyl und Alkoximinomethyl steht,

$R^3$ für Wasserstoff oder Alkyl steht,

$R^4$ für Alkyl, Alkenyl, Alkinyl, Halogenalkyl, Alkoxyalkyl sowie, falls $R^3$ für Alkyl steht, für die Gruppe $-SR^5$ steht,

$R^5$ für Alkyl, Halogenalkyl, gegebenenfalls substituiertes Phenyl, Alkoxycarbonyl, Dialkylamino steht, oder für den gleichen Rest steht, an den die Gruppierung $-SR^5$ gebunden ist, sowie für die Gruppierung $-NR^6-SO_2R^7$ steht, wobei

$R^6$ für Alkyl steht und

$R^7$ für Alkyl, Dialkylamino oder gegebenenfalls substituiertes Phenyl steht.

2)

Es wurde gefunden, daß die Oximcarbamate der Formel (I)

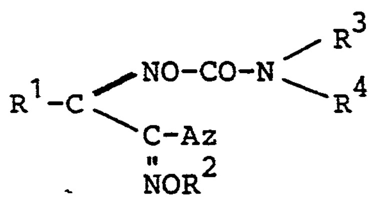

(I)

in welcher

Az für einen gegebenenfalls substituierten Azolylrest steht,

$R^1$ für gegebenenfalls substituiertes Alkyl steht,

$R^2$ für Alkyl, Halogenalkyl, Cycloalkyl, Alkenyl, Alkinyl, Alkyloxyalkyl, Alkylthioalkyl und Alkoximinomethyl steht,

$R^3$ für Wasserstoff oder Alkyl steht,

$R^4$ für Alkyl, Alkenyl, Alkinyl, Halogenalkyl, Alkoxyalkyl sowie, falls $R^3$ für Alkyl steht, für die Gruppe $-SR^5$ steht,

$R^5$ für Alkyl, Halogenalkyl, gegebenenfalls substituiertes Phenyl, Alkoxycarbonyl, Dialkylamino steht, oder für den gleichen Rest steht, an den die Gruppierung $-SR^5$ gebunden ist, sowie für die Gruppierung $-NR^6-SO_2R^7$ steht, wobei

$R^6$ für Alkyl steht und

$R^7$ für Alkyl, Dialkylamino oder gegebenenfalls substituiertes Phenyl steht;

hergestellt werden können, indem man

a) Oxime der Formel (II)

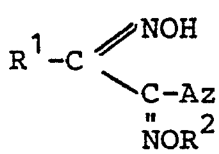

(II)

in welcher

Az, $R^1$ und $R^2$ die oben angegebenen Bedeutungen haben,

mit Isocyanaten der Formel (III)

$$R^4NCO \qquad (III)$$

in welcher
$R^4$ für Alkyl steht,

umsetzt oder

b) Oxime der Formel (II) mit Carbamoylhalogeniden der Formel (IV)

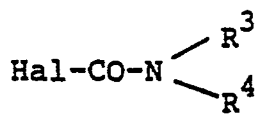

(IV)

in welcher
$R^3$ und $R^4$ die oben angegebene Bedeutung haben und
Hal für Fluor oder Chlor steht,

umsetzt, oder

c) Oxime der Formel (II) mit Phosgen und anschließend mit Aminen der Formel (V)

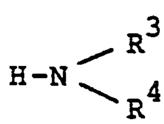

(V)

in welcher
$R^3$ und $R^4$ die oben angegebene Bedeutung haben,

umsetzt, oder

d) gegebenenfalls die nach Verfahren a), b) oder c) erhältlichen Oximcarbamate der Formel (VI)

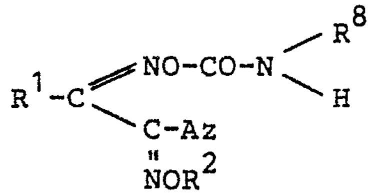

(VI)

in welcher
Az, $R^1$ und $R^2$ die oben angegebene Bedeutung haben und
$R^8$ für Alkyl steht,

mit Sulfenchloriden der Formel (VII)

$Cl-S-R^5$ (VII)

in welcher
$R^5$ die oben angegebene Bedeutung hat,

umsetzt.

3)

Es wurden die neuen Oxime der Formel (II) gefunden

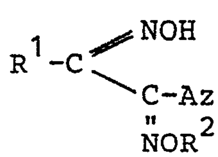

(II)

in welcher

Az, $R^1$ und $R^2$ die oben angegebene Bedeutung haben.

4)

Es wurde gefunden, daß die Oxime der Formel (II) hergestellt werden können, indem man Ketone der Formel (VIII)

$$R^1-\overset{O}{\overset{\|}{C}}-\underset{NOR^2}{\underset{\|}{C}}-Az \qquad (VIII)$$

mit Hydroxylamin oder seinen Salzen umsetzt.

Die erfindungsgemäßen Verbindungen der Formel (I) lassen sich als Insektizide, Akarizide und Nematizide verwenden. Sie zeigen dabei überraschenderweise bessere Wirkungen als die aus dem Stand der Technik bekannten Verbindungen.

Die erfindungsgemäßen Oximcarbamate sind durch die Formel (I) allgemein definiert. In dieser Formel steht Az vorzugsweise für die gegebenenfalls substituierten Azolyl-Reste Pyrazol-1-yl, Imidazol-1-yl, 1,2,4-Triazol-1-yl, 1,2,3-Triazol-1-yl, 1,3,4-Triazol-1-yl, wobei als Substituenten vorzugsweise in Frage kommen: Halogen, insbesondere Fluor, Chlor und Brom, Alkyl mit 1 bis 4 Kohlenstoffatomen, Halogenalkyl mit bis zu 2 Kohlenstoff- und bis zu 5 Halogenatomen, wie insbesondere Fluor- und Chloratomen, beispielsweise sei Trifluormethyl genannt, Alkoxy und Alkylthio mit jeweils bis zu 4 Kohlenstoffatomen und die Nitrogruppe.

$R^1$ steht vorzugsweise für gegebenenfalls ein- oder zweifach substituiertes Alkyl mit 1 bis 4 Kohlenstoffatomen, wobei als Substituenten vorzugsweise in Frage kommen: Halogen, insbesondere Fluor, Chlor oder Brom; Acyloxy, insbesondere Alkylcarbonyloxy mit 1 bis 4 Kohlenstoffatomen im Alkylteil; Hydroxy, Nitro, Cyano, sowie Alkoxy und Alkylthio mit jeweils 1 bis 4 Kohlenstoffatomen.

$R^2$ steht vorzugsweise für Alkyl mit 1 bis 4 C-Atomen, Alkenyl mit 2 bis 4 C-Atomen, Alkinyl mit 2 bis 4 C-Atomen, Halogenalkyl mit 1 bis 4 C-Atomen und bis zu 3 Halogenatomen, Cycloalkyl mit 3 bis 6 C-Atomen, Alkylthioalkyl mit 1 bis 2 C-Atomen in jedem Alkylteil sowie für Alkoxyiminomethyl mit 1 bis 4 C-Atomen im Alkyletherteil.

$R^3$ steht vorzugsweise für Wasserstoff oder geradkettiges oder verzweigtes Alkyl mit 1 bis 4 Kohlenstoffatomen.

$R^4$ steht vorzugsweise für geradkettiges oder verzweigtes Alkyl mit 1 bis 12 Kohlenstoffatomen, Alkenyl und Alkinyl mit jeweils 2 bis 4 Kohlenstoffatomen, ferner vorzugsweise für Halogenalkyl mit bis zu 2 Kohlenstoff- und bis zu 5 Halogenatomen, wie insbesondere Fluor- und Chloratomen, Halogenalkenyl mit 2 bis 4 Kohlenstoff- und bis zu 5 Halogenatomen, wie insbesondere Fluor- und Chloratomen, sowie vorzugsweise für Alkoxyalkyl mit bis zu 2 Kohlenstoffatomen in jedem Alkylteil.
$R^4$ steht außerdem vorzugsweise für die Gruppe $-S-R^5$, falls $R^3$ für Alkyl steht.

Dabei steht $R^5$ vorzugsweise für geradkettiges oder verzweigtes Alkyl mit 1 bis 4 Kohlenstoffatomen, Halogenalkyl mit 1 bis 2 Kohlenstoff- und bis zu 5 Halogenatomen, wie insbesondere Fluor- und Chloratomen, beispielsweise sei Trifluormethyl genannt, gegebenenfalls substituiertes Phenyl, wobei als Substituenten vorzugsweise genannt seien: Halogen, insbesondere Fluor, Chlor oder Brom, Alkyl mit 1 oder 2 Kohlenstoffatomen und Halogenalkyl mit 1 bis 2 Kohlenstoff- und bis zu 5 Halogenatomen, wie insbesondere Fluor- und Chloratomen, beispielsweise sei die Trifluormethylgruppe genannt, sowie vorzugsweise für Alkoxycarbonyl mit 1 bis 4 Kohlenstoffatomen im Alkoxyteil. $R^5$ steht ferner vorzugsweise für den gleichen Rest, an den die Gruppe $-S-R^5$ gebunden ist sowie für die Gruppe $-NR^6-SO_2R^7$, wobei $R^6$ vorzugsweise für Alkyl mit 1 bis 4 C-Atomen steht.

$R^7$ steht vorzugsweise für Alkyl mit 1 bis 4 C-Atomen, Dialkylamino mit jeweils 1 bis 4 C-Atomen in jedem Alkylteil sowie für gegebenenfalls substituiertes Phenyl, wobei als Substituenten vorzugsweise in Frage kommen: Halogen, insbesondere Fluor, Chlor oder Brom, Alkyl mit 1 bis 2 C-Atomen, Halogenalkyl mit 1 bis 2 C-Atomen und bis zu 5 Halogenatomen, wie insbesondere Fluor- und Chloratomen, beispielsweise sei Trifluormethyl genannt, Cyano und Nitro.

Ganz besonders bevorzugt sind diejenigen Oximcarbamate der Formel (I) in denen Az für Pyrazol-1-yl und 1,2,4-Triazol-1-yl steht;

$R^1$ für Methyl und gegebenenfalls substituiertes tertiäres Butyl steht, wobei als Substituenten genannt seien: Chlor, Fluor und Acetyloxy.

Ganz besonders bevorzugt steht $R^2$ für Methyl, Ethyl, Propyl, Allyl, Propargyl, Methylthiomethyl und Methoximinomethyl;

$R^3$ für Wasserstoff oder Methyl und

$R^4$ für Methyl oder die Gruppe $-S-R^5$, wobei $R^5$ für Trichlormethyl, Dichlorfluormethyl sowie für den gleichen Rest an den die Gruppierung $-S-R^5$ gebunden ist, steht.

Im einzelnen seien außer den Herstellungsbeispielen die folgenden Verbindungen genannt:

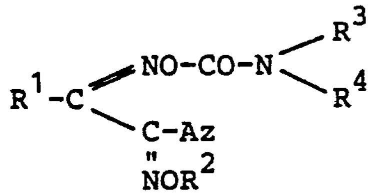

| Az | $R^1$ | $R^2$ | $R^3$ | $R^4$ |
|---|---|---|---|---|
| 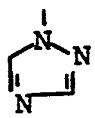 | 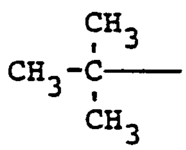 | $CH_3-$ | H | $CH_3$ |
| " | " | $C_2H_5-$ | H | $CH_3$ |
| " | " | $C_3H_7-$ | H | $CH_3$ |
| " | " | $-CH_2-CH=CH_2$ | H | $CH_3$ |
| " | " | $-CH_2-C\equiv CH$ | H | $CH_3$ |
| " | " | $-CH_2-S-CH_3$ | H | $CH_3$ |
| " | " | 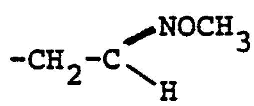 | H | $CH_3$ |
| " | " | $CH_3$ | $CH_3$ | -S- (dimer) |
| " | " | $C_2H_5$ | $CH_3$ | -S- (dimer) |

| Az | $R^1$ | $R^2$ | $R^3$ | $R^4$ |
|---|---|---|---|---|
| 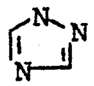 | 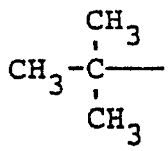 | $C_3H_7$ | $CH_3$ | -S- (dimer) |
| " | " | $-CH_2-CH=CH_2$ | $CH_3$ | -S- (dimer) |
| " | " | $-CH_2-C{\equiv}CH$ | $CH_3$ | -S- (dimer) |
| " | " | $-CH_2-S-CH_3$ | $CH_3$ | -S- (dimer) |
| " | " | 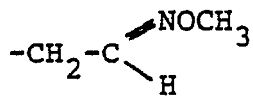 | $CH_3$ | -S- (dimer) |
| " | " | $CH_3$ | $CH_3$ | $SCCl_3$ |
| " | " | $C_2H_5$ | $CH_3$ | $SCCl_3$ |
| " | " | $C_3H_7$ | $CH_3$ | $SCCl_3$ |
| " | " | $-CH_2-CH=CH_2$ | $CH_3$ | $SCCl_3$ |
| " | " | $-CH_2-C{\equiv}CH$ | $CH_3$ | $SCCl_3$ |
| " | " | $-CH_2-S-CH_2$ | $CH_3$ | $SCCl_3$ |
| " | " | 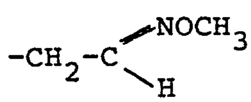 | $CH_3$ | $SCCl_3$ |
| " | " | $CH_3$ | $CH_3$ | $SCCl_2F$ |
| " | " | $C_2H_5$ | $CH_3$ | $SCCl_2F$ |

| Az | $R^1$ | $R^2$ | $R^3$ | $R^4$ |
|---|---|---|---|---|
| 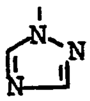 | 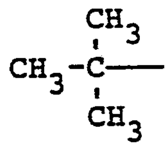 | $C_3H_7$ | $CH_3$ | $SCCl_2F$ |
| " | " | $-CH_2-CH=CH_2$ | $CH_3$ | $SCCl_2F$ |
| " | " | $-CH_2-C\equiv CH$ | $CH_3$ | $SCCl_2F$ |
| " | " | $-CH_2-S-CH_3$ | $CH_3$ | $SCCl_2F$ |
| " | " | 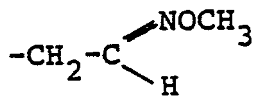 | $CH_3$ | $SCCl_2F$ |
| 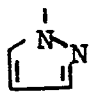 | " | $CH_3$ | H | $CH_3$ |
| " | " | $C_2H_5$ | H | $CH_3$ |
| " | " | $CH_3$ | $CH_3$ | -S- (dimer) |
| " | " | $C_2H_5$ | $CH_3$ | -S- (dimer) |
| " | " | $CH_3$ | $CH_3$ | $SCCl_3$ |
| " | " | $C_2H_5$ | $CH_3$ | $SCCl_3$ |
| " | " | $CH_3$ | $CH_3$ | $SCCl_2F$ |
| " | " | $C_2H_5$ | $CH_3$ | $SCCl_2F$ |

| Az | $R^1$ | $R^2$ | $R^3$ | $R^4$ |
|---|---|---|---|---|
| 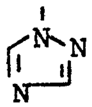 | 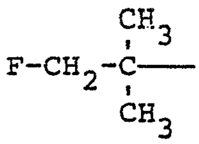 | $CH_3$ | H | $CH_3$ |
| " | " | $C_2H_5$ | H | $CH_3$ |
| " | " | $CH_3$ | $CH_3$ | -S- (dimer) |
| " | " | $C_2H_5$ | $CH_3$ | -S- (dimer) |
| " | " | $CH_3$ | $CH_3$ | $SCCl_3$ |
| " | " | $C_2H_5$ | $CH_3$ | $SCCl_3$ |
| " | " | $CH_3$ | $CH_3$ | $SCCl_2F$ |
| " | " | $C_2H_5$ | $CH_3$ | $SCCl_2F$ |
| " | 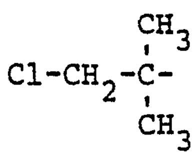 | $CH_3$ | H | $CH_3$ |
| " | " | $C_2H_5$ | H | $CH_3$ |
| " | " | $CH_3$ | $CH_3$ | -S- (dimer) |
| " | " | $C_2H_5$ | $CH_3$ | -S- (dimer) |
| " | " | $CH_3$ | $CH_3$ | $SCCl_3$ |
| " | " | $C_2H_5$ | $CH_3$ | $SCCl_3$ |
| " | " | $CH_3$ | $CH_3$ | $SCCl_2F$ |
| " | " | $C_2H_5$ | $CH_3$ | $SCCl_2F$ |

| Az | $R^1$ | $R^2$ | $R^3$ | $R^4$ |
|---|---|---|---|---|
| 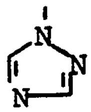 | 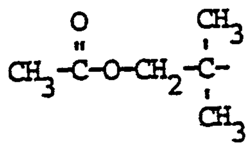 | $CH_3$ | H | $CH_3$ |
| " | " | $C_2H_5$ | H | $CH_3$ |
| " | " | $CH_3$ | $CH_3$ | -S- (dimer) |
| " | " | $C_2H_5$ | $CH_3$ | -S- (dimer) |
| " | " | $CH_3$ | $CH_3$ | $-SCCl_3$ |
| " | " | $C_2H_5$ | $CH_3$ | $-SCCl_3$ |
| " | " | $CH_3$ | $CH_3$ | $-SCCl_2F$ |
| " | " | $C_2H_5$ | $CH_3$ | $-SCCl_2F$ |
| " | $CH_3-$ | $CH_3-$ | H | $CH_3$ |
| " | $CH_3-$ | $C_2H_5$ | H | $CH_3$ |
| " | $CH_3-$ | $C_3H_7$ | H | $CH_3$ |
| " | $CH_3-$ | $-CH_2-CH=CH_2$ | H | $CH_3$ |
| " | $CH_3-$ | $-CH_2-C\equiv CH$ | H | $CH_3$ |
| " | $CH_3-$ | $CH_3$ | $CH_3$ | -S- (dimer) |
| " | $CH_3-$ | $C_2H_5$ | $CH_3$ | -S- (dimer) |
| " | $CH_3-$ | $CH_3$ | $CH_3$ | $-SCCl_3$ |
| " | $CH_3-$ | $C_2H_5$ | $CH_3$ | $-SCCl_3$ |
| " | $CH_3-$ | $CH_3$ | $CH_3$ | $-SCCl_2F$ |
| " | $CH_3-$ | $C_2H_5$ | $CH_3$ | $-SCCl_2F$ |

Verwendet man beispielsweise 3,3-Dimethyl-2-oximino-1-methoximino-1-(1,2,4-triazol-1-yl)-butan und Methylisocyanat als Ausgangsstoffe, so kann der Reaktionsablauf durch das folgende Formelschema wiedergegeben werden (Verfahren a)):

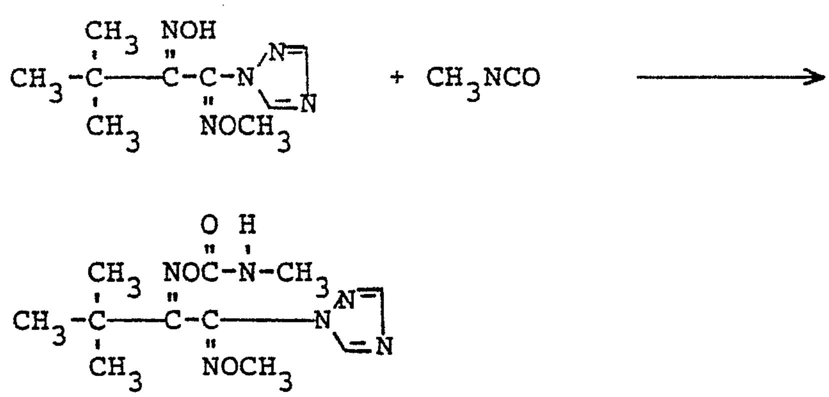

Verwendet man beispielsweise 2-Oximino-1-methoximino-1-(1,2,4-triazol-1-yl)-butan und N,N'-Bis-(fluorcarbonyl)-thio-bis-methylamin als Ausgangsstoffe, so kann der Reaktionsablauf durch das folgende Formelschema wiedergegeben werden (Verfahren b)):

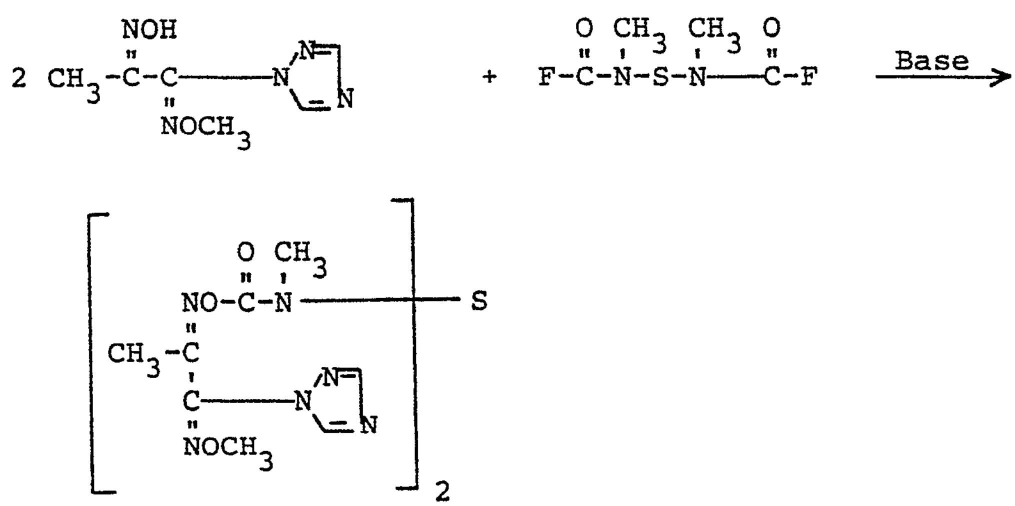

Verwendet man 3,3-Dimethyl-2-oximino-1-ethoximino-1-(1,2,4-triazol-1-yl)-butan, Phosgen und Dimethylamin als Ausgangsstoffe, so kann der Reaktionsablauf durch das folgende Formelschema wiedergegeben werden (Verfahren c)):

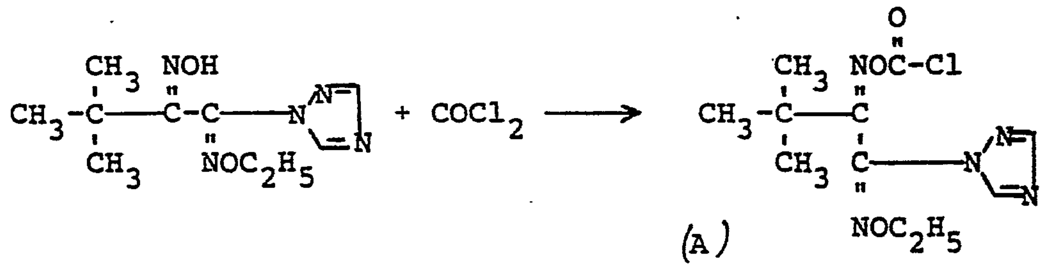

(A)

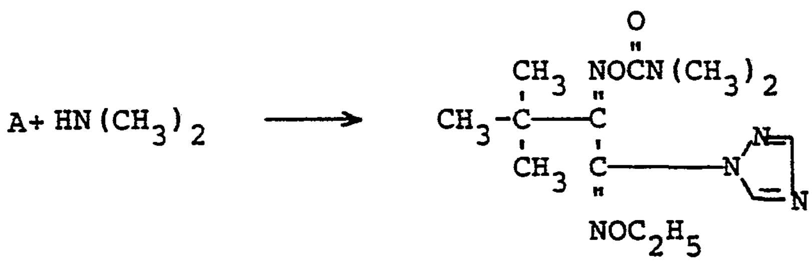

Verwendet man 3,3-Dimethyl-2-methylcarbamoyloximino-1-methoximino-1-(1,2,4-triazol-1-yl)-butan und Chlorphenyl-sulfenyl-chlorid als Ausgangsstoffe, so kann der Reaktionsablauf durch das folgende Formelschema wiedergegeben werden (Verfahren d)):

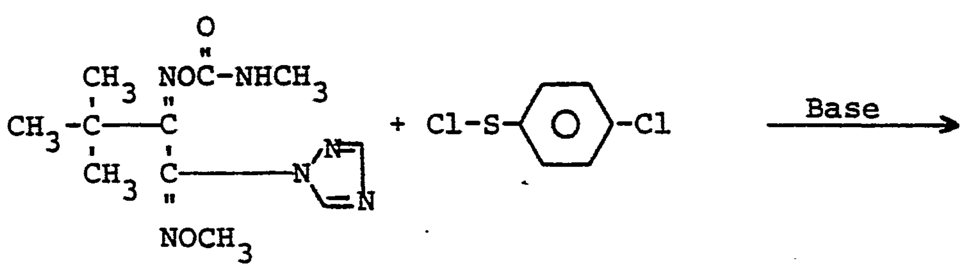


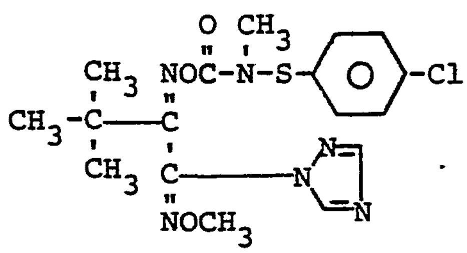

Als Verdünnungsmittel kommen für die Umsetzung gemäß Verfahrensvariante (a), (b), (c) und (d) vorzugsweise alle inerten organischen Lösungsmittel in Frage. Hierzu gehören vorzugsweise Ketone, wie Diethylketon, insbesondere Aceton und Methylethylketon; Nitrile, wie Propionitril, insbesondere Acetonitril; Ether, wie Tetrahydrofuran oder Dioxan; Formamide, wie insbesondere Dimethylformamid und halogenierte Kohlenwasserstoffe, wie Methylenchlorid, Tetrachlorkohlenstoff oder Chloroform. Bei Verwendung von Natriumhydrid als Hilfsstoff werden vorzugsweise polare organische Lösungsmittel verwendet, wie insbesondere Hexamethylphosphorsäuretriamid.

Wird die Umsetzung nach Verfahren (b), (c) und (d) in Gegenwart eines Säurebinders vorgenommen, so kann man alle üblicherweise verwendbaren anorganischen und organischen Säurebinder zugeben. Hierzu gehören vorzugsweise Natriumcarbonat, Kaliumcarbonat und Natriumhydrogencarbonat, ferner niedere tertiäre Alkylamine, Cycloalkylamine oder Arylalkylamine, wie beispielsweise Triethylamin, N,N-Dimethyl-benzylamin, Dicyclohexylamin, weiterhin Pyridin und Diazabicyclooctan.

Die Reaktionstemperaturen können bei der Durchführung des Verfahrens (b) in einem größeren Bereich variiert werden. Im allgemeinen arbeitet man zwischen 0 und 100°C, vorzugsweise zwischen 10 und 80°C.

Bei der Durchführung des Verfahrens (b) setzt man vorzugsweise auf 1 Mol der Verbindung der Formel (II) 1

bis 2 Mol, für den Fall eines dimeren Produktes 0,5 Mol, an Carbamoylchlorid der Formel (IV) und 1 bis 2 Mol Säurebinder ein. Die Isolierung der Verbindungen der Formel (I) erfolgt in allgemein üblicher und bekannter Weise.

Als Katalysatoren können beim Verfahren (a) vorzugsweise verwendet werden:

tertiäre Basen, wie Triethylamin, Pyridin, Zinn-organische Verbindungen, wie Dibutylzinndilaurat.

Die Reaktionstemperaturen können bei der Durchführung des Verfahrens (a) in einem größeren Bereich variiert werden. Im allgemeinen arbeitet man zwischen 0 und 100°C, vorzugsweise zwischen 20 und 85°C.

Bei der Durchführung des Verfahrens (a) setzt man auf 1 Mol der Verbindung der Formel (II) 1 bis 2 Mol Isocyanat der Formel (III) ein. Zur Isolierung der Verbindungen der Formel (I) wird das Lösungsmittel abdestilliert und der Rückstand nach üblichen Methoden aufgearbeitet.

Die Reaktionstemperaturen können bei der Durchführung des Verfahrens (c) in einem größeren Bereich variiert werden. Im allgemeinen arbeitet man zwischen 0 und 100°C, vorzugsweise zwischen 0 und 85°C.

Bei der Durchführung des Verfahrens (c) setzt man vorzugsweise auf 1 Mol der Verbindung der Formel (II) 1

bis 1,5 Mol Phosgen und 1 bis 1,5 Mol an Amin der Formel (V) ein. Es hat sich als vorteilhaft erwiesen, den Säurebinder in geringem Überschuß (bis ca. 30 Gew.-%) und gegebenenfalls das Natriumhydrid in einem Überschuß bis zu ca. 50 Gew.-% einzusetzen. Die Isolierung der Verbindungen der Formel (I) erfolgt in üblicher Weise.

Die Reaktionstemperaturen können bei der Durchführung des Verfahrens (d) in einem größeren Bereich variiert werden. Im allgemeinen arbeitet man zwischen 0 und 100°C, vorzugsweise zwischen 10 und 50°C.

Bei der Durchführung des Verfahrens (d) werden die Ausgangsstoffe vorzugsweise in molaren Mengen eingesetzt. Die Isolierung der Verbindungen der Formel (I) erfolgt nach üblichen Methoden.

Die für die Verfahren (a), (b) und (c) als Ausgangsstoffe zu verwendenden Oxime sind durch die Formel (II) allgemein definiert. In dieser Formel stehen Az, $R^1$ und $R^2$ vorzugsweise für die Reste, die die bei den Verbindungen der Formel (I) angegebene bevorzugte und besonders bevorzugte Bedeutung besitzen.

Die Oxime der Formel (II) sind noch nicht bekannt und können hergestellt werden, indem man Ketone der Formel (VIII)

$$R^1-\overset{O}{\overset{\|}{C}}-\underset{\underset{NOR^2}{\|}}{C}-Az \qquad (VIII)$$

in welcher

Az, $R^1$ und $R^2$ die oben angegebene Bedeutung haben,

mit Hydroxylamin in Gegenwart eines Lösungsmittels, vorzugsweise Alkohols bzw. wäßrigen Alkohols bei Temperaturen vorzugsweise zwischen 50 und 80°C umsetzt. Dabei wird das Hydroxylamin vorzugsweise in Form seiner Salze, insbesondere als Hydrochlorid, in Gegenwart einer Hilfsbase, wie beispielsweise Natriumacetat, eingesetzt. Die Isolierung der Verbindungen der Formel (II) erfolgt dadurch, daß man das während der Umsetzung entstehende Produkt nach Abdestillieren des Lösungsmittels nach üblichen Methoden aufarbeitet.

Als Ausgangsstoffe der Formel (II) seien beispielsweise genannt:

3,3-Dimethyl-2-oximino-1-methoximino-1-(1,2,4-triazol-1-yl)-butan

3,3-Dimethyl-2-oximino-1-ethoximino-1-(1,2,4-triazol-1-yl)-butan

3,3-Dimethyl-2-oximino-1-propoximino-1-(1,2,4-triazol-1-yl)-butan.

Die weiterhin für die erfindungsgemäße Umsetzung (a) als Ausgangsstoffe benötigten Isocyanate sind durch die Formel (III) allgemein definiert. In dieser Formel steht $R^4$ vorzugsweise für geradkettiges oder verzweigtes Alkyl mit 1 bis 12 Kohlenstoffatomen.

Die Isocyanate der Formel (III) sind bekannt oder lassen sich nach allgemein üblichen und bekannten Verfahren herstellen, z.B. durch Umsetzung von Aminen mit Phosgen und anschließendem Erhitzen. Diese Verfahren sind aus den allgemeinen Lehrbüchern der organischen Chemie bekannt.

Als Ausgangsstoffe der Formel (III) seien beispielsweise genannt:

Methylisocyanat, Ethylisocyanat, i-Propylisocyanat, t-Butylisocyanat, Heptylisocyanat, Dodecylisocyanat.

Die für die erfindungsgemäße Umsetzung (b) als Ausgangsstoffe benötigten Carbamoylhalogenide sind durch die Formel (IV) allgemein definiert. In dieser Formel stehen $R^3$ und $R^4$ vorzugsweise für die Reste, die bei den Verbindungen der Formel (I) bereits vorzugsweise genannt wurden.

Die Carbamoylhalogenide der Formel (IV) sind bekannt oder lassen sich nach allgemein üblichen und bekannten Verfahren herstellen, z.B. erhält man sie durch Umsetzung von Aminen mit Phosgen (diese Verfahren sind aus den allgemeinen Lehrbüchern der organischen Chemie bekannt) oder durch Umsetzung der entsprechenden Carbamidsäurehalogenide mit entsprechenden Sulfenchloriden (vgl. hierzu auch die Angaben in DE-AS 1 297 096, DE-OS 2 357 930 und 2 409 463 sowie US-Patentschrift 3 939 192).

Als Ausgangsstoffe der Formel (IV) seien beispielsweise genannt:

Dimethylcarbamoylchlorid, Methylethylcarbamoylchlorid, Allylmethylcarbamoylchlorid, Methoxymethyl-methylcarbamoylchlorid, Methyl-trifluormethylcarbamoylchlorid, Ethylvinylcarbamoylchlorid, N,N'-Bis-(fluorcarbonyl)-thio-bis-methylamin, N-Methyl-N-trichlormethylsulfenyl-carbamidsäurefluorid, N-Methyl-N-fluordichlormethyl-sulfenyl-carbamidsäurefluorid, N-Methyl-N-chlordifluormethylsulfenyl-carbamidsäurefluorid, N-Methyl-N-(3-trifluormethylphenyl)-sulfenyl-carbamidsäurefluorid, N-Methyl-N-(methoxycarbonyl-sulfenyl)-carbamidsäurefluorid, N-Methyl-N-[(3-methylphenyl-sulfonyl)-methyl-amino-sulfenyl)]-carbamidsäurefluorid, N-Methyl-N-[(4-chlorphenyl)-sulfenyl]-carbamidsäurefluorid, N-Methyl-N-[(4-methylphenyl-sulfonyl)-methylamino-sulfenyl]-carbamidsäurefluorid und die entsprechenden Carbamidsäure-chloride.

Die für die erfindungsgemäße Umsetzung (c) als Ausgangsstoffe zu verwendenden Amine sind durch die Formel (V) allgemein definiert. In dieser Formel stehen $R^3$ und $R^4$ vorzugsweise für die Reste, die bei den Verbindungen der Formel (I) bereits vorzugsweise genannt wurden.

Die Amine der Formel (V) sind allgemein bekannte Verbindungen. Als Beispiele seien genannt:

Ammoniak, Methylamin, Ethylamin, Dimethylamin, Methylethylamin, Allylmethylamin, Methoxymethyl-methylamin, Methyl-trifluormethylamin, Ethylvinylamin.

Die für die erfindungsgemäße Umsetzung (d) als Ausgangsstoffe zu verwendenden Sulfenchloride sind durch die Formel (VII) allgemein definiert. In dieser Formel steht $R^5$ vorzugsweise für die Reste, die bei den Verbindungen der Formel (I) bereits vorzugsweise genannt wurden.

Die Sulfenchloride der Formel (VII) sind allgemein bekannte Verbindungen der organischen Chemie. Als Beispiele seien genannt:

Trichlormethylsulfenchlorid, Dichlorfluormethylsulfenchlorid, Chlordifluormethylsulfenchlorid, Trifluormethylsulfenchlorid, Phenylsulfenchlorid, 2,4-Dichlorphenylsulfenchlorid, 3-Trifluormethylsulfenchlorid, 3-Methylphenylsulfenchlorid, Methylsulfenylchlorid, 4-Chlor-3-trifluor-methylphenylsulfenylchlorid, Methoxycarbonylsulfenylchlorid, Ethoxycarbonylsulfenylchlorid.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hygienesektor vorkommen.Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophagus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips femoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Tineola bisselliella, Tinea pellionella, Hofmannophila pseudospretella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolantha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp.
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xyphinema spp., Trichodorus spp., Globodera spp.

Die erfindungsgemäßen Wirkstoffe können in ihren handelsüblichen Formulierungen sowie in den aus diesen Formulierungen bereiteten Anwendungsformen in Mischung mit anderen Wirkstoffen, wie Insektiziden, Lockstoffen, Sterilantien, Akariziden, Nematiziden, Fungiziden, wachstumsregulierenden Stoffen oder Herbiziden vorliegen. Zu den Insektiziden zählen beispielsweise Phosphorsäureester, Carbamate, Carbonsäureester, chlorierte Kohlenwasserstoffe, Phenylharnstoffe, durch Mikroorganismen hergestellte Stoffe u.a.

Die erfindungsgemäßen Wirkstoffe können ferner in ihren handelsüblichen Formulierungen sowie in den aus diesen Formulierungen bereiteten Anwendungsformen in Mischung mit Synergisten vorliegen. Synergisten sind Verbindungen, durch die die Wirkung der Wirkstoffe gesteigert wird, ohne daß der zugesetzte Synergist selbst aktiv wirksam sein muß.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,0001 und 1 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Herstellungsbeispiele

Beispiel 1

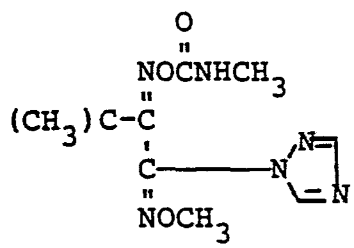

2,25 g (0,01 Mol) 3,3-Dimethyl-2-oximino-1-methoximino-1-(1,2,4-triazol-1-yl)-butan gelöst in 120 ml abs. Aceton werden unter Rühren mit 2,28 g (0,04 Mol) Methylisocyanat versetzt. Man läßt 10 h nachrühren, engt die Reaktionsmischung ein, nimmt den Rückstand in Methylenchlorid auf und wäscht zweimal mit Wasser. Die org. Phase wird über $Na_2SO_4$ getrocknet, abfiltriert und eingeengt.

Ausbeute: 2,5 g (89 % der Theorie), Fp. 127°C.

Beispiel 2

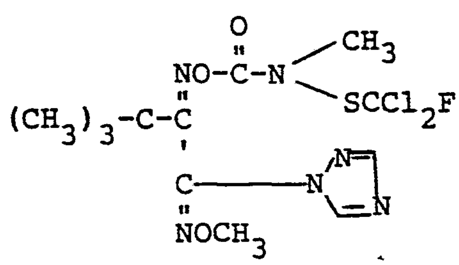

2,0 g (0,009 Mol) 3,3-Dimethyl-2-oximino-1-methoximino-1-(1,2,4-triazol-1-yl)-butan und 1,6 g (0,009 Mol) N-Dichlorfluormethansulfenyl-N-methylcarbamidsäurefluorid

werden in 50 ml absolutem Methylenchlorid gelöst und unter Rühren tropfenweise mit 1 g (0,01 Mol) Triethylamin versetzt. Es wird 6 Stunden bei Raumtemperatur gerührt und dann 2-mal mit Wasser gewaschen. Nach dem Trocknen über $Na_2SO_4$ wird das Lösungsmittel im Vakuum entfernt.

Ausbeute: 2,5 g Öl (71 % der Theorie), $n_D^{20}$ 1.5127.

Beispiel 3

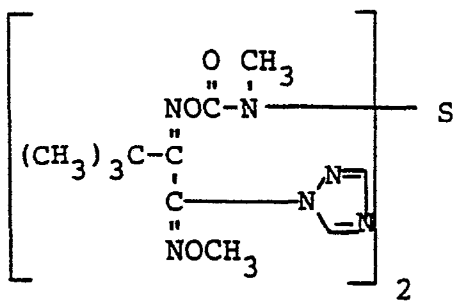

4,0 g (0,018 Mol) 3,3-Dimethyl-2-oximino-1-methoximino-1-(1,2,4-triazol-1-yl)-butan und 1,64 g (0,009 Mol) N,N'-Bis-(fluorcarbonyl)-thio-bis-methylamin werden in 50 ml absolutem Methylenchlorid unter Rühren bei Raumtemperatur mit 2 g (0,02 Mol) Triethylamin versetzt. Es wird 6 Stunden bei Raumtemperatur gerührt und dann 2-mal mit Wasser gewaschen. Nach dem Trocknen über $Na_2SO_4$ wird das Lösungsmittel im Vakuum entfernt.

Ausbeute: 3,9 g (71 % der Theorie), Fp. 60°C.

Analog werden die folgenden Beispiele erhalten:

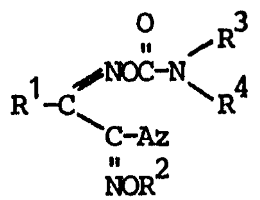 (V)

| Bsp.-Nr. | Az | $R^1$ | $R^2$ | $R^3$ | $R^4$ | Fp. (°C) bzw. Brechungsindex |
|---|---|---|---|---|---|---|
| 4 | 1,2,4-Triazol | $(CH_3)_3C$ | $CH_3$ | $CH_3$ | $-SCCl_3$ | 1.5135 |
| 5 | Pyrazol | $(CH_3)_3C$ | $CH_3$ | H | $CH_3$ | 115 |
| 6 | Pyrazol | $(CH_3)_3C$ | $CH_3$ | $CH_3$ | -S- (dimer) | 100-105 |
| 7 | 1,2,4-Triazol | $(CH_3)_3C$ | $C_2H_5$ | H | $CH_3$ | 111 |
| 8 | 1,2,4-Triazol | $(CH_3)_3C$ | $C_2H_5$ | $CH_3$ | -S- (dimer) | 1.5109 |
| 9 | 1,2,4-Triazol | $(CH_3)_3C$ | $C_2H_5$ | $CH_3$ | $-SCCl_2F$ | 1.5080 |
| 10 | 1,2,4-Triazol | $(CH_3)_3C$ | $C_3H_7$ | H | $CH_3$ | 93-96 |

Fortsetzung:

| Bsp.-Nr. | Az | $R^1$ | $R^2$ | $R^3$ | $R^4$ | Fp. (°C) bzw. Brechungsindex |
|---|---|---|---|---|---|---|
| 11 | 1,2,4-Triazol | $(CH_3)_3C$ | $C_3H_7$ | $CH_3$ | -S- (dimer) | 1.5126 |
| 12 | 1,2,4-Triazol | $(CH_3)_3C$ | $C_3H_7$ | $CH_3$ | $SCCl_3$ | 1.5234 |
| 13 | 1,2,4-Triazol | $(CH_3)_3C$ | $C_3H_7$ | $CH_3$ | $SCCl_2F$ | 1.5088 |
| 14 | 1,2,4-Triazol | $(CH_3)_3C$ | $C_4H_9$ | H | $CH_3$ | 1.4985 |
| 15 | 1,2,4-Triazol | $(CH_3)_3C$ | $-CH_2-CH=CH_2$ | H | $CH_3$ | halbkristallin |
| 16 | 1,2,4-Triazol | $(CH_3)_3C$ | $-CH_2-C\equiv CH$ | H | $CH_3$ | zähes Öl |
| 17 | 1,2,4-Triazol | $(CH_3)_3C$ | $-CH_2-S-CH_3$ | H | $CH_3$ | 100-108 |
| 18 | 1,2,4-Triazol | $CH_3$ | $CH_3$ | H | $CH_3$ | 120-124 |
| 19 | 1,2,4-Triazol | $CH_3$ | $C_2H_5$ | H | $CH_3$ | 96 |
| 20 | 1,2,4-Triazol | $(CH_3)_3C$ | iso-$C_3H_7$ | H | $CH_3$ | 95 |

Herstellung der Vorprodukte

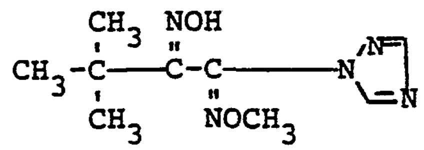

42 g (0,2 Mol) 3,3-Dimethyl-1-methoximino-1-(1,2,4-triazol-1-yl)-butan-2-on, 41,7 g (0,6 Mol) Hydroxylaminhydrochlorid, 49,2 g (0,6 Mol) Natriumacetat, 450 ml Ethanol und 600 ml Wasser werden 20 h am Rückfluß gekocht. Die Reaktionsmischung wird eingeengt, in verdünnter KOH-Lösung aufgenommen und mit Chloroform gewaschen. Die wäßrige Phase wird mit verdünnter Salzsäure auf pH 5 gebracht, das ausgefallene Produkt abgenutscht und mit Wasser gewaschen.

Ausbeute: 23 g (51 % der Theorie), Fp. 137°C.

Beispiel A

Phaedon-Larven-Test

Lösungsmittel: 3 Gewichtsteile Dimethylformamid
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Meerrettichblattkäfer-Larven (Phaedon cochleariae) besetzt, solange die Blätter noch feucht sind.

Nach der gewünschten Zeit wird die Abtötung in % bestimmt. Dabei bedeutet 100 %, daß alle Käfer-Larven abgetötet wurden; 0 % bedeutet, daß keine Käfer-Larven abgetötet wurden.

Bei diesem Test zeigen z.B. die folgenden Verbindungen der Herstellungsbeispiele überlegene Wirksamkeit gegenüber dem Stand der Technik:

1, 3

Beispiel B

Plutella-Test

Lösungsmittel: 3 Gewichtsteile Dimethylformamid
Emulgator : 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik:

5, 7, 15, 16, 4, 12, 13, 6

Beispiel C

Tetranychus-Test (resistent)

| | | |
|---|---|---|
| Lösungsmittel: | 3 Gewichtsteile | Dimethylformamid |
| Emulgator: | 1 Gewichtsteil | Alkylarylpolyglykoläther |

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 1

7, 15, 16, 12, 2, 13

0083744

Beispiel D

Grenzkonzentrations-Test / Wurzelsystemische Wirkung

Testinsekt: Myzus persicae
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik:

1, 3, 2, 4, 16, 17

Beispiel E

Grenzkonzentrations-Test / Wurzelsystemische Wirkung

Testinsekt: Phaedon cochleariae-Larven
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt. Dabei spielt die Konzentration des Wirkstoffs in der Zubereitung praktisch keine Rolle, entscheidend ist allein die Wirkstoffgewichtsmenge pro Volumeneinheit Boden, welche in ppm (=mg/l) angegeben wird. Man füllt den behandelten Boden in Töpfe und bepflanzt diese mit Kohl (Brassica oleracea). Der Wirkstoff kann so von den Pflanzenwurzeln aus dem Boden aufgenommen und in die Blätter transportiert werden.

Für den Nachweis des wurzelsystemischen Effektes werden nach 7 Tagen ausschließlich die Blätter mit den obengenannten Testtieren besetzt. Nach weiteren 2 Tagen erfolgt die Auswertung durch Zählen oder Schätzen der toten Tiere. Aus den Abtötungszahlen wird die wurzelsystemische Wirkung des Wirkstoffs abgeleitet. Sie ist 100 %, wenn alle Testtiere abgetötet sind und 0 %, wenn noch genau so viele Testinsekten leben wie bei der unbehandelten Kontrolle.

Bei diesem Test zeigen z.B. die folgenden Verbindungen der Herstellungsbeispiele überlegene Wirkung gegenüber dem Stand der Technik: 1, 3, 2, 7, 15

Beispiel F

Grenzkonzentrations-Test / Nematoden

Testnematode: Meloidogyne incognita
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27°C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele überlegene Wirksamkeit gegenüber dem Stand der Technik:

1, 3, 2, 4, 7, 10, 15, 16

0083744

## Patentansprüche

1) Oximcarbamate der Formel (I)

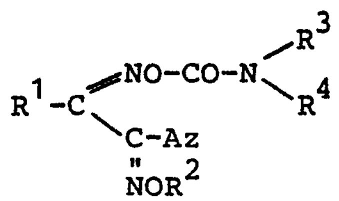

(I)

in welcher

Az für einen gegebenenfalls substituierten Azolylrest steht,

$R^1$ für gegebenenfalls substituiertes Alkyl steht,

$R^2$ für Alkyl, Halogenalkyl, Cycloalkyl, Alkenyl, Alkinyl, Alkyloxyalkyl, Alkylthioalkyl und Alkoximinomethyl steht,

$R^3$ für Wasserstoff oder Alkyl steht,

$R^4$ für Alkyl, Alkenyl, Alkinyl, Halogenalkyl, Alkoxyalkyl sowie, falls $R^3$ für Alkyl steht, für die Gruppe $-SR^5$ steht,

$R^5$ für Alkyl, Halogenalkyl, gegebenenfalls substituiertes Phenyl, Alkoxycarbonyl, Dialkylamino steht, oder für den gleichen Rest steht, an den die Gruppierung $-SR^5$ gebunden ist, sowie für die Gruppierung $-NR^6-SO_2R^7$ steht, wobei

$R^6$ für Alkyl steht und

$R^7$ für Alkyl, Dialkylamino oder gegebenenfalls substituiertes Phenyl steht.

2) Verfahren zur Herstellung der Oximcarbamate der Formel (I)

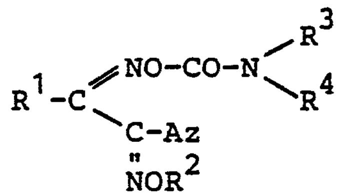

(I)

in welcher

Az für einen gegebenenfalls substituierten Azolylrest steht,

$R^1$ für gegebenenfalls substituiertes Alkyl steht,

$R^2$ für Alkyl, Halogenalkyl, Cycloalkyl, Alkenyl, Alkinyl, Alkyloxyalkyl, Alkylthioalkyl und Alkoximinomethyl steht,

$R^3$ für Wasserstoff oder Alkyl steht,

$R^4$ für Alkyl, Alkenyl, Alkinyl, Halogenalkyl, Alkoxyalkyl sowie, falls $R^3$ für Alkyl steht, für die Gruppe $-SR^5$ steht,

$R^5$ für Alkyl, Halogenalkyl, gegebenenfalls substituiertes Phenyl, Alkoxycarbonyl, Dialkylamino steht, oder für den gleichen Rest steht, an den die Gruppierung $-SR^5$ gebunden ist, sowie für die Gruppierung $-NR^6-SO_2R^7$ steht, wobei

$R^6$ für Alkyl steht und

$R^7$ für Alkyl, Dialkylamino oder gegebenenfalls substituiertes Phenyl steht;

indem man

a) Oxime der Formel (II)

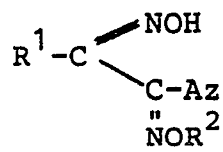

(II)

in welcher
Az, $R^1$ und $R^2$ die oben angegebenen Bedeutungen haben,

mit Isocyanaten der Formel (III)

$R^4NCO$ (III)

in welcher
$R^4$ für Alkyl steht,

umsetzt oder

b) Oxime der Formel (II) mit Carbamoylhalogeniden der Formel (IV)

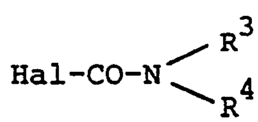

(IV)

in welcher

$R^3$ und $R^4$ die oben angegebene Bedeutung haben und
Hal für Fluor oder Chlor steht,

umsetzt, oder

c) Oxime der Formel (II) mit Phosgen und anschließend mit Aminen der Formel (V)

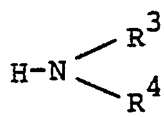

(V)

in welcher
$R^3$ und $R^4$ die oben angegebene Bedeutung haben,

umsetzt, oder

d) gegebenenfalls die nach Verfahren a), b) oder c) erhältlichen Oximcarbamate der Formel (VI)

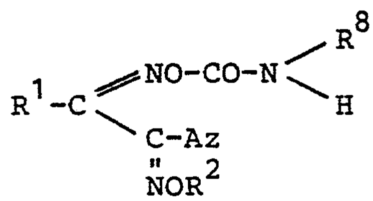

(VI)

in welcher
Az, $R^1$ und $R^2$ die oben angegebene Bedeutung haben und
$R^8$ für Alkyl steht,

mit Sulfenchloriden der Formel (VII)

$$Cl-S-R^5 \qquad (VII)$$

in welcher
$R^5$ die oben angegebene Bedeutung hat,

umsetzt.

3) Oxime der Formel (II)

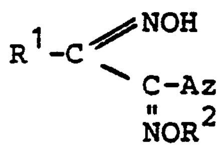

(II)

in welcher
Az für einen gegebenenfalls substituierten Azolylrest steht,
$R^1$ für gegebenenfalls substituiertes Alkyl steht,
$R^2$ für Alkyl, Halogenalkyl, Cycloalkyl, Alkenyl, Alkinyl, Alkyloxyalkyl, Alkylthioalkyl und Alkoximinomethyl steht.

4) Verfahren zur Herstellung der Oxime der Formel (II)

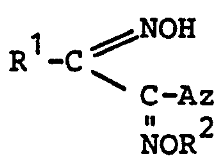

(II)

in welcher

Az für einen gegebenenfalls substituierten Azolylrest steht,

$R^1$ für gegebenenfalls substituiertes Alkyl steht,

$R^2$ für Alkyl, Halogenalkyl, Cycloalkyl, Alkenyl, Alkinyl, Alkyloxyalkyl, Alkylthioalkyl und Alkoximinomethyl steht,

indem man Ketone der Formel (VIII)

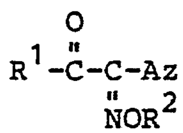

(VIII)

in welcher

$A_2$, $R^1$ und $R^2$ die oben angegebene Bedeutung hat, mit Hydroxylamin oder seinen Salzen umsetzt.

5) Schädlingsbekämpfungsmittel, gekennzeichnet durch einen Gehalt an mindestens einem Oximcarbamat der Formel (I).

6) Verwendung von Oximcarbamaten der Formel (I) zur Bekämpfung von Schädlingen.

7) Verfahren zur Bekämpfung von Schädlingen, dadurch gekennzeichnet, daß man Oximcarbamate der Formel (I) auf Schädlinge und/oder ihren Lebensraum einwirken läßt.

8) Verfahren zur Herstellung von Schädlingsbekämpfungsmitteln, dadurch gekennzeichnet, daß man Oximcarbamate der Formel (I) mit Streckmitteln und/oder oberflächenaktiven Mitteln vermischt.